# EUROPEAN PATENT SPECIFICATION

(11) **EP 3 299 819 B1**
(45) Date of publication and mention of the grant of the patent: **10.03.2021**
(21) Application number: 17192503.5
(22) Date of filing: 22.09.2017
(51) Int. Cl.: G01N 33/68

(54) **METHOD AND APPARATUS FOR ASSISTING DIAGNOSIS OF RISK OF PROGRESSION TO STAGE 2 OR HIGHER DIABETIC NEPHROPATHY**
VERFAHREN UND VORRICHTUNG ZUR UNTERSTÜTZUNG DER DIAGNOSE DES RISIKOS DES FORTSCHREITENS VON DIABETISCHER NEPHROPATHIE ZU STUFE 2 ODER HÖHER
PROCÉDÉ ET APPAREIL POUR FACILITER LE DIAGNOSTIC DE RISQUE DE PROGRESSION VERS LE STADE 2 OU PLUS DE LA NÉPHROPATHIE DIABÉTIQUE

(30) Priority: 23.09.2016 JP 2016185652
(43) Date of publication of application: 28.03.2018
(73) Proprietor: SYSMEX CORPORATION, Kobe-shi Hyogo 651-0073 (JP)
(72) Inventor: Shirahase, Yasushi, Kobe-shi, Hyogo, 651-0073 (JP); Yoshida, Toshiyuki, Kobe-shi, Hyogo, 651-0073 (JP)
(74) Representative: De Clercq & Partners

(56) References cited:
- EP-A1- 2 442 106
- Yan Ling ET AL: "Circulating ApoE Level is Independently Associated with Urinary Albumin Excretion in Type 2 Diabetic Patients", Internal Medicine, 1 January 2011 (2011-01-01), pages 2961-2966, XP055425784, Japan DOI: 10.2169/internalmedicine.50.6216 Retrieved from the Internet: URL:https://www.jstage.jst.go.jp/article/i nternalmedicine/50/24/50_24_2961/_pdf [retrieved on 2017-11-16]
- VIRGINIA URQUIDI ET AL: "Diagnostic Potential of Urinary [alpha]1-Antitrypsin and Apolipoprotein E in the Detection of Bladder Cancer", JOURNAL OF UROLOGY., vol. 188, no. 6, 1 December 2012 (2012-12-01), pages 2377-2383, XP055425583, BALTIMORE, MD, US ISSN: 0022-5347, DOI: 10.1016/j.juro.2012.07.094
- TEMESGEN FISEHA: "Urinary biomarkers for early diabetic nephropathy in type 2 diabetic patients", BIOMARKER RESEARCH, vol. 3, no. 1, 4 July 2015 (2015-07-04), XP055425980, DOI: 10.1186/s40364-015-0042-3
- Karen S L Lam ET AL: "Nephrology Dialysis Transplantation Apolipoprotein (a) levels and phenotypes in NIDDM patients with microalbuminuria and albuminuria", Nephrol Dial Transplant, 1 January 1996 (1996-01-01), pages 2229-2236, XP55426438, Retrieved from the Internet: URL:https://www.google.com/url?sa=t&rct=j& q=&esrc=s&source=web&cd=7&ved=0ahUKEwjWn-H It8PXAhVEXhoKHRwdD6MQFgg-MAY&url=https://a cademic.oup.com/ndt/article-pdf/11/11/2229 /6900474/11-11-2229.pdf&usg=AOvVaw3w7AVpw1 4Ocqhu5RKXfK89 [retrieved on 2017-11-16]
- SIMA HASHEMIPOUR ET AL: "Urinary Total Protein as the Predictor of Albuminuria in Diabetic Patients", INTERNATIONAL JOURNAL OF ENDOCRINOLOGY & METABOLISM, vol. 10, no. 3, 1 June 2012 (2012-06-01), pages 523-526, XP055426442, ISSN: 1726-913X, DOI: 10.5812/ijem.4236
- Y G Wan ET AL: "Characteristic of urinary protein spectrum in patients with stage III diabetic nephropathy and its regression analysis with traditional Chinese medicine symptom].", , 1 January 2013 (2013-01-01), pages 4157-4163, XP055426444, Retrieved from the Internet: URL:http://www.ncbi.nlm.nih.gov/pubmed/247 91509 [retrieved on 2017-11-17]
- RAO PATURI V ET AL: "Proteomic identification of urinary biomarkers of diabetic nephropathy", DIABETES CARE, AMERICAN DIABETES ASSOCIATION, ALEXANDRIA, VA, US, vol. 30, no. 3, 1 March 2007 (2007-03-01), pages 629-637, XP002492989, ISSN: 0149-5992, DOI: 10.2337/DC06-2056
- M L Alter ET AL: "Early urinary and plasma biomarkers for experimental diabetic nephropathy.", , 1 January 2012 (2012-01-01), pages 659-671, XP055426448, Retrieved from the Internet: URL:http://www.ncbi.nlm.nih.gov/pubmed/229 97966 [retrieved on 2017-11-17]
- H Mulec ET AL: "Cholesterol: a renal risk factor in diabetic nephropathy?", , 1 January 1993 (1993-01-01), pages 196-201, XP055426451, Retrieved from the Internet: URL:http://www.ncbi.nlm.nih.gov/pubmed/832 2783 [retrieved on 2017-11-17]
- NICOLAE M. PANDURU ET AL: "Kidney Injury Molecule-1 and the Loss of Kidney Function in Diabetic Nephropathy: A Likely Causal Link in Patients With Type 1 Diabetes", DIABETES CARE, vol. 38, no. 6, 17 March 2015 (2015-03-17) , pages 1130-1137, XP055426425, US ISSN: 0149-5992, DOI: 10.2337/dc14-2330

## Description

### TECHNICAL FIELD

The present invention relates to a method and apparatus for assisting diagnosis of risk of progression from stage 1 diabetic nephropathy to stage 2 or higher diabetic nephropathy.

### BACKGROUND

Diabetic nephropathy is one of complications of diabetes mellitus and is a kidney disease which progresses through different stages. The stages of diabetic nephropathy are classified from stages 1 to 5. As diabetic nephropathy progresses, it will eventually become kidney failure, requiring dialysis treatment. Dialysis treatment is a heavy burden for patients. In recent years, the number of patients who need dialysis treatment has increased, and the increase in medical expenses accompanying this has become a problem.

In diabetic nephropathy, renal function can be restored when proper treatment can be started at an early stage. However, at a more advanced stage, although treatment can delay the progression of the disease, it is difficult to maintain and restore renal function. Therefore, finding a patient at risk of progressing diabetic nephropathy at an early stage where recovery of renal function can be expected and aggressively treating the patient can alleviate the future burden of the patient. It also contributes greatly to the reduction of medical expenses.

Currently, biomarkers useful for the diagnosis of diabetic nephropathy are being investigated. For example, Yamato K. et al., (2004, Diabetes Res Clin Pract., 69(2):124-8) discloses that the amount of secretion of apolipoprotein E (ApoE) of macrophages decreases in patients with diabetes mellitus type II. Ilhan N. et al.,(2007, Cell Biochem Funct. 25(5):527-32) shows that polymorphism of epsilon 4 allele of ApoE is associated with diabetic nephropathy and the risk of diabetic nephropathy. Ling et al. (2011, Internal Medicine 50:2961-2966) report that serum ApoE levels were correlated with urinary albumin excretion in type 2 diabetic patients, and that the urinary albumin to creatinine ratio can be used to classify type 2 diabetic patients into a normoalbuminuric or an albuminuric group. EP2442106 is directed to a test method of diabetic nephropathy, including the staging of diabetic nephropathy based on the detection of urinary podocalyxin. Fiseha et al. (2015, Biomarker Research 3:16) reviews urinary biomarkers, not including urinary ApoE, for detecting diabetic nephropathy in type 2 diabetic patients. Lan et al. (1996, Nephrol Dial Transplant 11:2229-2236) compared plasma apolipoprotein A levels between non-insulin-dependent diabetes mellitus (NIDDM) patients with normoalbuminuria, microalbuminuria or albuminuria to determine its usefulness to evaluate the risk of cardiovascular mortality in NIDDM patients. Hashemipour et al. (2012, Int J Endocrinol Metab. 10:523-526) describes the diagnosis of micro- and macroalbuminuria in diabetic patients based on urinary total protein. Rao et al. (2007, Diabetes Care 30:629-637) shows that proteomic analysis allows to identify urinary biomarkers for diabetic nephropathy and for staging diabetic nephropathy (e.g. urine proteins were identified that were differentially expressed in type 2 diabetic patients with macroalbuminuria compared with type 2 diabetes without albuminuria). Urinary ApoE was not identified. Alter et al. (2012, Clin. Lab. 58:659-671) discloses plasma and urinary biomarkers, not including urinary ApoE, for the diagnosis of diabetic nephropathy. Wan et al. (2013, China Journal of Chinese Materia Medica 38) studied urinary proteins, not including urinary ApoE, in patients with stage III diabetic nephropathy. Mulec et al. (1993, Journal of Kidney Diseases 22:196-201) studied the relationship between serum cholesterol, triglycerides, apolipoprotein AI and B, and lipoprotein(a), but not urinary ApoE levels, and the rate of decline in glomerular filtration rate in patients with type 1 diabetes and nephropathy. Pandaru et al. (2015, Diabetes Care 38:1130-1137) studied the predictive value of urinary kidney injury molecule (KIM)-1 for progression of diabetic nephropathy in type 1 diabetes.
Urquidi et al. (2012, J Urol. 188:2377-2383) describes the diagnostic potential of urinary ApoE in the detection of bladder cancer.

In neither Yamato K. et al., (2004, above) nor Ilhan N, et al. (2007, above), the relationship between urinary ApoE concentration and diabetic nephropathy is described or suggested. On the other hand, as described above, it is important to find a patient with a high risk of progression at an early stage and start treatment. Therefore, development of a means enabling diagnosis of such risk is desired.

### SUMMARY OF THE INVENTION

The present invention provides a method for assisting the diagnosis of the risk of progression to stage 2 or higher diabetic nephropathy, comprising measuring the urinary apolipoprotein E (ApoE) concentration and optionally the urinary concentration of a nephropathy marker other than the urinary ApoE of a diabetic patient with stage 1 diabetic nephropathy, and determining whether the patient has a high risk of progressing to stage 2 or higher diabetic nephropathy based on a result of the measurement step, wherein the patient is determined to have a high risk of progressing to stage 2 or higher diabetic nephropathy when:
- the value of urinary ApoE concentration is less than a predetermined threshold value, or
- the ratio of the value of urinary ApoE concentration to the value of the urinary concentration of said other nephropathy marker is less than a predetermined threshold value, or
- the ratio of the value of the urinary concentration of said other nephropathy marker to the value of urinary ApoE concentration is equal to or greater than the predetermined threshold value; and wherein the patient is determined to have a low risk of progressing to stage 2 or higher diabetic nephropathy when:
   - the value of urinary ApoE concentration is equal to or greater than a predetermined threshold value, or
   - the ratio of the value of urinary ApoE concentration to the value of the urinary concentration of said other nephropathy marker is equal to or greater than a predetermined threshold value, or
   - the ratio of the value of the urinary concentration of said other nephropathy marker to the value of urinary ApoE concentration is less than the predetermined threshold value.
In particular embodiments, said other nephropathy marker is selected from the group consisting of uTP, uALB, α1-M, NAG, L-FABP, NGAL, KIM- 1, uCHO, type IV collagen, ApoA1 and ApoB.

The present invention also provides an apparatus for acquiring data for assisting the diagnosis of the risk of progression to stage 2 or higher diabetic nephropathy, comprising a computer containing a processor and a memory under control of the processor, wherein the memory stores a computer program for making the computer execute the following steps: measuring the urinary apolipoprotein E (ApoE) concentration of a diabetic patient with stage 1 diabetic nephropathy and optionally the urinary concentration of a nephropathy marker other than the urinary ApoE, and determining whether the patient has a high risk of progressing to stage 2 or higher diabetic nephropathy based on a result of the measurement step,
wherein the patient is determined to have a high risk of progressing to stage 2 or higher diabetic nephropathy when:
- the value of urinary ApoE concentration is less than a predetermined threshold value, or
- the ratio of the value of urinary ApoE concentration to the value of the urinary concentration of said other nephropathy marker is less than a predetermined threshold value, or
- the ratio of the value of the urinary concentration of said other nephropathy marker to the value of urinary ApoE concentration is equal to or greater than the predetermined threshold value; and wherein the patient is determined to have a low risk of progressing to stage 2 or higher diabetic nephropathy when:
   - the value of urinary ApoE concentration is equal to or greater than a predetermined threshold value, or
   - the ratio of the value of urinary ApoE concentration to the value of the urinary concentration of said other nephropathy marker is equal to or greater than a predetermined threshold value, or
   - the ratio of the value of the urinary concentration of said other nephropathy marker to the value of urinary ApoE concentration is less than the predetermined threshold value.

According to the present invention, it becomes possible to assist diagnosis of risk that a patient with stage 1 diabetic nephropathy progresses to stage 2 or higher diabetic nephropathy.

### BRIEF DESCRIPTION OF THE DRAWINGS

Fig. 1A is a view showing an example of an appearance of a reagent kit as described herein.
Fig. 1B is a view showing an example of an appearance of a reagent kit as described herein.
Fig. 1C is a view showing an example of an appearance of a reagent kit as described herein.
Fig. ID is a view showing an example of an appearance of a reagent kit as described herein.
Fig. IE is a view showing an example of an appearance of a reagent kit as described herein.
Fig. IF is a view showing an example of an appearance of a reagent kit as described herein.
Fig. 2 is a schematic view showing an example of a progression risk diagnosis assisting apparatus.
Fig. 3 is a block diagram showing a functional configuration of the progression risk diagnosis assisting apparatus in Fig. 2.
Fig. 4 is a block diagram showing a hardware configuration of the progression risk diagnosis assisting apparatus shown in Fig. 2.
Fig. 5A is an example of a flowchart of determination for acquiring data to assist diagnosis of risk of progression to stage 2 or higher diabetic nephropathy, using the progression risk diagnosis assisting apparatus shown in Fig. 2.
Fig. 5B is an example of a flowchart of determination for acquiring data to assist diagnosis of risk of progression to stage 2 or higher diabetic nephropathy, using the progression risk diagnosis assisting apparatus shown in Fig. 2.

### DETAILED DESCRIPTION OF THE PREFERRED EMBODIMENTS

In the diagnostic methods of the present invention, a diabetic patient (hereinafter, also referred to as "subject") with stage 1 diabetic nephropathy is to be diagnosed. Stage 1 diabetic nephropathy refers to a stage in which the albuminuria categories in the severity classification of chronic kidney disease (CKD) described in KDIGO (Kidney Disease: Improving Global Outcomes) 2012 Clinical Practice Guideline for the Evaluation and Management of Chronic Kidney Disease is A1, and the GFR classification is G1, G2, G3a or G3b. A diabetic patient with stage 1 diabetic nephropathy is a patient diagnosed as diabetes mellitus, having a urinary albumin/creatinine (Cr) ratio of lower than 30 mg/gCr and an estimated GFR of 30 mL/min/1.73 m² or more. Stage 1 diabetic nephropathy is said to be early stage of nephropathy, medical findings of nephropathy are not observed, and there is no subjective symptom. The diagnosis method for diabetes mellitus is not particularly limited, and a known method may be used for diagnosis. Methods for measuring urinary albumin and creatinine and a method for calculating estimated GFR are known in the art.

In the methods provided herein, urine collected from a subject is used as a sample. The nature of the urine collected from the subject is not particularly limited and may depend on the physical condition, the presence or absence of meal and medication of the subject, and the like. The timing of urine collection is also not particularly limited, and any one of early morning urine, occasional urine and pooled urine may be used. In the methods of the present invention, the urine in an amount of about 100 to 500 µL is sufficient.

As necessary, pretreatment may be performed on urine collected from a subject so as to be suitable for the measurement of ApoE concentration. Examples of such pretreatment include dilution, pH adjustment and the like. For example, it is possible to dilute urine and adjust the pH by mixing the urine with a buffer having a predetermined pH. The buffer is not particularly limited as long as it has a buffering action in a pH range suitable for the measurement of ApoE concentration. Examples thereof include Good's buffers such as Tris, MES, PIPES, TES and HEPES, phosphate buffered saline (PBS), and the like. When amorphous salts are present in urine, a chelating agent such as EDTA salt may be added to the urine to remove them. Hereinafter, urine collected from a subject and pretreated urine are also referred to as "urine sample".

In the urine of a diabetic patient with stage 1 diabetic nephropathy, ApoE is thought to be derived from lipoproteins taken up by phagocytes such as macrophages or produced by macrophages themselves. In particular embodiments, ApoE may be released into urine from phagocytes incorporating the ApoE as a result of pretreatment of the urine. The method of releasing ApoE from cells is not particularly limited, and examples thereof include solubilization with a surfactant, ultrasonic disruption, freeze-thawing, and the like. Solubilization of cells with a surfactant can be carried out, for example, by mixing urine collected from a subject and a buffer containing a surfactant. The amount of the buffer containing a surfactant to be added can be appropriately set, but it may be added, for example, in an amount of between 1 time or more and 50 times or less, and preferably between 1 time or more and 10 times or less the urine volume. The type of the surfactant is not particularly limited as long as it does not disturb the measurement of ApoE. Examples thereof include Triton X-100, Tween 20, octyl glucoside, and the like. The final concentration of the surfactant in the urine sample can be appropriately set according to the type of the surfactant. For example, when using Triton X-100, the final concentration in the urine sample is between 0.001% (v/v) or more and 10% (v/v) or less, and preferably between 0.01% (v/v) or more and 1% (v/v) or less.

The urinary ApoE concentration can be measured by a method that can acquire a value reflecting the concentration or amount of ApoE (hereinafter, also referred to as "measured value of ApoE") contained in the urine sample. As such a method, a method of capturing ApoE using a capture body that specifically binds to ApoE is preferable. The measured value of ApoE can be acquired by detecting the ApoE captured by the capture body by a known method. Examples of the capture body that specifically binds to ApoE include antibodies, aptamers, and the like. The antibody is particularly preferable among them. The antibody may be any of monoclonal antibodies, polyclonal antibodies, and fragments thereof (for example, Fab, F(ab')2, etc.). The antibody that specifically binds to ApoE (hereinafter, also referred to as "anti-ApoE antibody") itself is known in the art. The anti-ApoE antibody is generally available.

In particular embodiments, it is preferable to measure the urinary ApoE concentration by an immunoassay using an anti-ApoE antibody. Examples of immunoassays include enzyme-linked immunosorbent assay (ELISA method), an immune complex metastasis measurement method (see JP H01-254868 A), immunoturbidimetry, immunochromatography, latex agglutination method, and the like. Commercially available assay kits such as an ELISA kit may be used. As an example of the measurement process, the case of measuring the urinary ApoE concentration by a sandwich ELISA method will be described below.

First, a complex containing urinary ApoE, an antibody for capturing ApoE (hereinafter, also referred to as "capture antibody") and an antibody for detecting ApoE (hereinafter, also referred to as "detection antibody") is formed on a solid phase. This complex can be formed by mixing a urine sample, a capture antibody and a detection antibody. Then, a solution containing the complex is brought into contact with a solid phase capable of capturing the capture antibody, whereby the complex can be formed on a solid phase. Alternatively, a solid phase previously immobilized with a capture antibody may be used. That is, a solid phase immobilized with a capture antibody, a urine sample, and a detection antibody are brought into contact with each other, whereby the complex can be formed on a solid phase. When both the capture antibody and the detection antibody are monoclonal antibodies, it is preferable that the epitopes be different from each other.

The mode of immobilization of the capture antibody on the solid phase is not particularly limited. For example, the capture antibody and the solid phase may be bound directly, or the capture antibody and the solid phase may be indirectly bound via another substance. Examples of the direct bond include physical adsorption and the like. Examples of the indirect bond include a bond via a combination of biotin and avidin or streptavidin (hereinafter, also referred to as "avidins"). In this case, by preliminarily modifying the capture antibody with biotin and previously binding avidins to the solid phase, the capture antibody and the solid phase can be indirectly bound via the bond between the biotin and the avidins.

The material of the solid phase is not particularly limited, and it can be selected from, for example, organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, etc.), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include particles, membranes, microplates, microtubes, test tubes, and the like.

The measured value of ApoE in urine can be acquired by detecting the complex formed on the solid phase by a method known in the art. For example, when an antibody labeled with a labeling substance is used as a detection antibody, the measured value of ApoE can be acquired by detecting a signal generated by the labeling substance. Alternatively, also when a labeled secondary antibody against the detection antibody is used, the measured value of ApoE can be acquired in the same manner.

In particular embodiments, a B/F (Bound/Free) separation for removing an unreacted free component not forming a complex may be performed between the process of forming the complex and the process of detecting the complex. The unreacted free component refers to a component not constituting a complex. Examples thereof include capture antibodies not bound to ApoE, detection antibodies, and the like. The means of B/F separation is not particularly limited, and when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particles with a magnet. After removing the unreacted free component, the solid phase capturing the complex may be washed with a suitable aqueous medium such as PBS.

The phrase "detecting a signal" herein includes qualitatively detecting the presence or absence of a signal, quantifying a signal intensity, and semi-quantitatively detecting the intensity of a signal. Semi-quantitative detection means to show the intensity of the signal in stages such as "no signal generated", "weak", "medium", "strong", and the like. In particular embodiments, it is preferable to detect the intensity of a signal quantitatively or semi-quantitatively.

The labeling substance is not particularly limited as long as a detectable signal is generated. For example, it may be a substance which itself generates a signal (hereinafter, also referred to as "signal generating substance") or a substance which catalyzes the reaction of other substances to generate a signal. Examples of the signal generating substance include fluorescent substances, radioactive isotopes, and the like. Examples of the substance that catalyzes the reaction of other substances to generate a detectable signal include enzymes and the like. Examples of the enzymes include alkaline phosphatase, peroxidase, β-galactosidase, luciferase, and the like. Examples of the fluorescent substances include fluorescent dyes such as fluorescein isothiocyanate (FITC), rhodamine and Alexa Fluor (registered trademark), fluorescent proteins such as GFP, and the like. Examples of the radioisotopes include ¹²⁵I, ¹⁴C, ¹²P, and the like. Among them, an enzyme is preferable as a labeling substance, and alkaline phosphatase and peroxidase are particularly preferable.

Methods for detecting a signal are known in the art. In methods of the invention, a measurement method may be appropriately selected according to the type of signal derived from the labeling substance. For example, when the labeling substance is an enzyme, signals such as light and color generated by reacting a substrate for the enzyme can be measured by using a known apparatus such as a spectrophotometer.

The substrate of the enzyme can be appropriately selected from known substrates according to the type of the enzyme. For example, when alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate. When peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB).

When the labeling substance is a radioactive isotope, radiation as a signal can be measured using a known apparatus such as a scintillation counter. When the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used.

The detection result of the signal may be used as the measured value of ApoE. For example, when quantitatively detecting the intensity of a signal, the signal intensity value itself or a value acquired from the intensity value can be used as the measured value of ApoE. Examples of the value acquired from the signal intensity value include a value obtained by subtracting the signal intensity value of a negative control sample or a background value from the signal intensity value of ApoE. The negative control sample can be appropriately selected, and examples thereof include urine obtained from a healthy subject and the like.

In particular embodiments, the measured value of ApoE may be acquired for a plurality of standard samples whose ApoE concentrations are known, and a calibration curve showing the relationship between the ApoE concentration and the measured value of ApoE may be prepared. The value of urinary ApoE concentration can be acquired by applying the measured value of ApoE acquired from the urine sample to this calibration curve. In order to avoid an error due to urine volume, it is preferable to correct the value of the acquired urinary ApoE concentration by the value of the urinary creatinine concentration of the subject. Specifically, it is preferable to convert the value of urinary ApoE concentration to the ApoE concentration (µg/gCr) per gram of creatinine. This makes it possible to compare the values of urinary ApoE concentration between specimens.

In particular embodiments, the urinary ApoE concentration may be measured by a sandwich ELISA method using a capture antibody immobilized on magnetic particles and a detection antibody labeled with a labeling substance. In this case, measurement may be carried out using a commercially available fully automated immunoassay system such as HISCL series (manufactured by Sysmex Corporation).

Optionally, the urinary concentrations of other nephropathy markers are measured, in addition to the measurement of the urinary ApoE concentration as described above, to acquire ratios of the value of the urinary ApoE concentration and the values of the urinary concentrations of other nephropathy markers, and may be used for determination of the risk of progression to stage 2 or higher diabetic nephropathy as described later. The above ratios may be the ratio of the value of urinary ApoE concentration to the value of the urinary concentrations of other nephropathy markers or may be the ratio of the values of the urinary concentrations of other nephropathy markers to the value of urinary ApoE concentration. The "ratio" herein is synonymous with the ratio value. For example, the "ratio of A to B" means "A/B".

The nature of the other nephropathy marker is not particularly limited, and it is preferably selected from the group consisting of urinary protein (uTP), urinary albumin (uALB), α1-microglobulin (α1M), N-acetylglucosaminidase (NAG), L-type fatty acid binding protein (LFABP), lipocalin-2 (NGAL), Kidney injury molecule-1 (KIM-1), urinary cholesterol (uCHO), type IV collagen, apolipoprotein A1 (ApoA1) and apolipoprotein B (ApoB), and more preferably selected from the group consisting of uALB, NAG, NGAL, KIM-1, uCHO and ApoA1.

The concentrations of other nephropathy markers can be measured according to a method known to those skilled in the art. The concentrations of other nephropathy markers can be measured, for example, in the same manner as described above for ApoE. In the same manner as described above for ApoE, the urine collected from a subject may be pretreated so as to be suitable for the measurement of the concentrations of other nephropathy markers.

Next, in the methods of the invention, it is determined whether or not the risk that the diabetic patient with stage 1 diabetic nephropathy as the subject progresses to stage 2 or higher diabetic nephropathy is high, based on the value of urinary ApoE concentration. Specifically, when the value of urinary ApoE concentration is less than a predetermined threshold value, it is determined that the risk of the subject progressing to stage 2 or higher diabetic nephropathy is high (that is, can be determined to be in a high-risk group). When the value of urinary ApoE concentration is equal to or greater than the predetermined threshold value, it is determined that the risk of the subject progressing to stage 2 or higher diabetic nephropathy is not high or is low (that is, can be determined to be in a low-risk group). Diabetic nephropathy is known not only in cases progressing in the order of stage 1, stage 2, stage 3 and stage 4, but also in cases progressing from stage 1 to stage 4 without going through stage 2 and stage 3. In the former cases, as the urinary albumin/Cr ratio rises, the stage progresses from stage 1 to stage 2 or higher. In the latter cases, the GFR classification worsens, and the stage progresses from stage 1 to stage 4. The method of the invention may be used as a method of determining the risk of progression from stage 1 diabetic nephropathy to stage 2 or stage 4. In these embodiments, the risk of progression from stage 1 diabetic nephropathy to stage 2 or stage 4 is determined in the determination process.

In the case where it has been determined that the risk of progression to stage 2 or higher diabetic nephropathy is high by the method of the present invention, when the subject does not receive treatment, it may be predicted that the subject will progress to stage 2 or higher diabetic nephropathy within a predetermined period, or it may be predicted that renal function will decline from half a year to a year after urine collection. In the case where it has been determined that the risk of progression to stage 2 or higher diabetic nephropathy is low, even when the subject does not receive treatment, it may be predicted that the renal function will not decline within a predetermined period, and the stage will not proceed. The predetermined period is, for example, a period of 12 months, and preferably 6 months. As described above, the method of the present invention makes it possible to provide a doctor and the like with information that assists diagnosis of risk of progression to stage 2 or higher diabetic nephropathy. "Evidence-based Practice Guideline for the Treatment for Diabetes in Japan 2013" (The Japan Diabetes Society) teaches strict blood glucose control and blood pressure control as prevention of the onset of nephropathy. When a subject has transitioned to stage 2 diabetic nephropathy showing microalbuminuria, the guideline teaches to treat with medicine by an angiotensin converting enzyme (ACE) inhibitor and an angiotensin II receptor antagonist. According to the present disclosure, a diabetic patient diagnosed as having a high progression risk at stage 1 diabetic nephropathy can be administered with an ACE inhibitor and an angiotensin II receptor antagonist at an early stage, whereby progression to stage 2 or higher diabetic nephropathy can be prevented.

The above-mentioned predetermined threshold value is not particularly limited, and it can be appropriately set. For example, the predetermined threshold value may be empirically set by accumulating data on the renal function including the value of urinary ApoE concentration for diabetic patients with stage 1 diabetic nephropathy. Alternatively, the predetermined threshold value may be set as follows. First, urine is collected from a plurality of diabetic patients with stage 1 diabetic nephropathy, and the urinary ApoE concentration is measured. After a lapse of a predetermined period from the collection of urine, these patients are examined for renal function, and the stage of diabetic nephropathy is confirmed. The measured urinary ApoE concentration data is classified into data of a patient group whose renal function has declined and data of a patient group whose renal function has not declined. Then, a value that can most accurately distinguish the urinary ApoE concentration of the patient group whose renal function has declined and the urinary ApoE concentration of the patient group whose renal function has not declined is obtained, and the value is set as the predetermined threshold value. In setting the threshold value, it is preferable to consider sensitivity, specificity, positive predictive value, negative predictive value, and the like.

The risk of progression to stage 2 or higher diabetic nephropathy can be also determined, based on the ratios of the value of the urinary ApoE concentration acquired as described above and the values of the urinary concentrations of other nephropathy markers. In the case where the above ratios are the ratios of the value of urinary ApoE concentration to the values of the urinary concentrations of other nephropathy markers, when the ratios are less than the predetermined threshold value, it is determined that the risk for the subject of progressing to stage 2 or higher diabetic nephropathy is high, and when the ratios are equal to or greater than the predetermined threshold value, it is determined that the risk for the subject of progressing to stage 2 or higher diabetic nephropathy is not high or is low. On the other hand, in the case where the above ratios are the ratios of the values of the urinary concentrations of other nephropathy markers to the value of urinary ApoE concentration, when the ratios are equal to or greater than the predetermined threshold value, it is determined that the risk for the subject of progressing to stage 2 or higher diabetic nephropathy is high, and when the ratios are less than the predetermined threshold value, it is determined that the risk for the subject of progressing to stage 2 or higher diabetic nephropathy is not high or is low. The setting of the threshold value can be carried out in the same manner as described above for the determination based on the urinary ApoE concentration.

The present disclosure also includes a reagent kit used for the method of assisting diagnosis of the risk of progression to stage 2 or higher diabetic nephropathy. That is, the present disclosure provides a reagent kit for diagnosing the risk of progression to stage 2 or higher diabetic nephropathy in a diabetic patient with stage 1 diabetic nephropathy (hereinafter, also referred to as "reagent kit"), comprising a first capture body that specifically binds to ApoE for capturing ApoE, a second capture body that specifically binds to ApoE for detecting ApoE, and a labeling substance. When the labeling substance is an enzyme, the reagent kit may further contain a substrate for the enzyme.The kits may further comprise instructions for carrying out the methods according to the invention.

In the kits provided herein, the forms of the first capture body, the second capture body, the labeling substance and the substrate are not particularly limited, and they may be a solid (for example, powder, crystal, freeze-dried product, etc.) or liquid (for example, solution, suspension, emulsion, etc.). It is preferable that the first capture body, the second capture body, the labeling substance and the substrate be stored in separate containers or individually packaged. The details of the urine sample, the first capture body, the second capture body, the labeling substance and the substrate are the same as those described in the explanation of the diagnosis assisting method above. It is preferable that both the first capture body and the second capture body be anti-ApoE antibodies. When both the first capture body and the second capture body are monoclonal antibodies, it is preferable that the epitopes are different from each other.

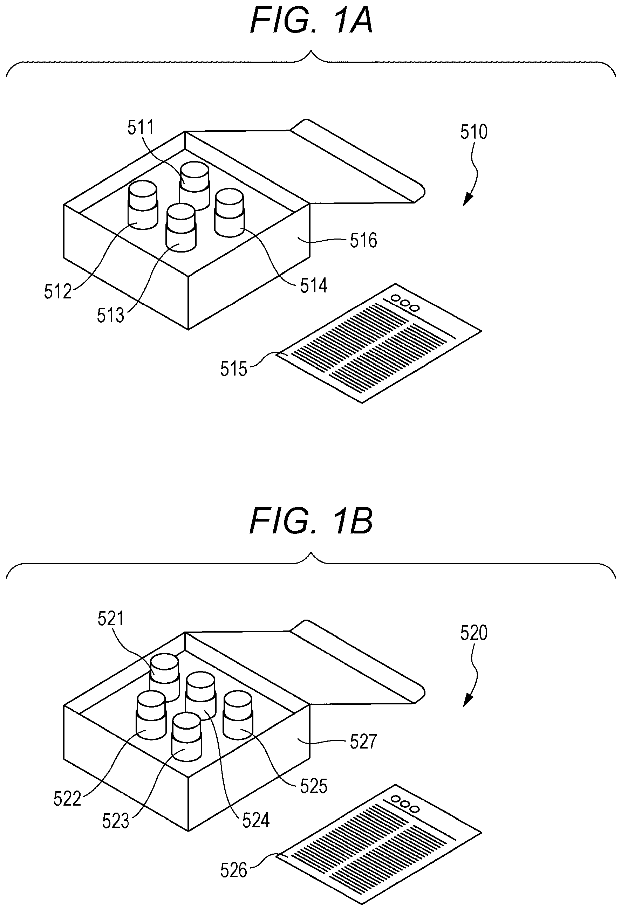

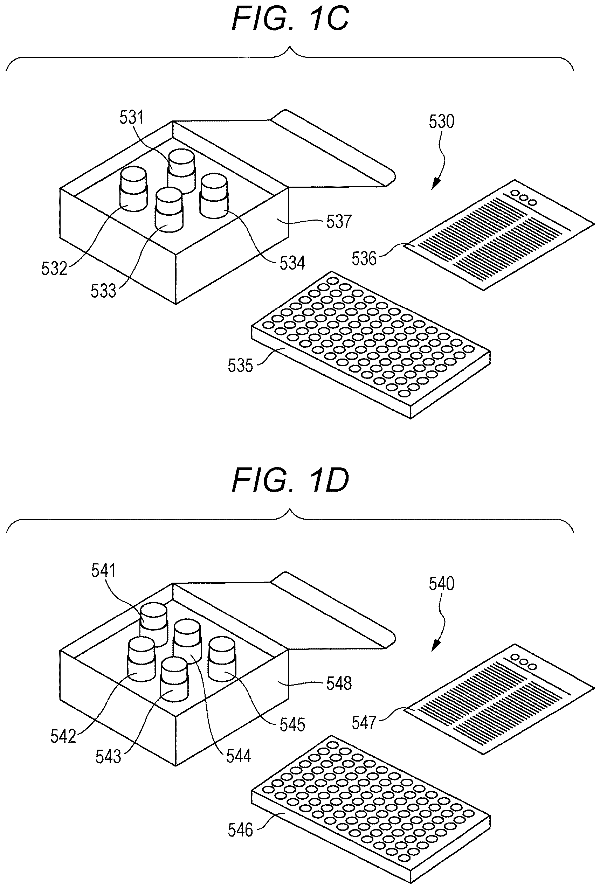

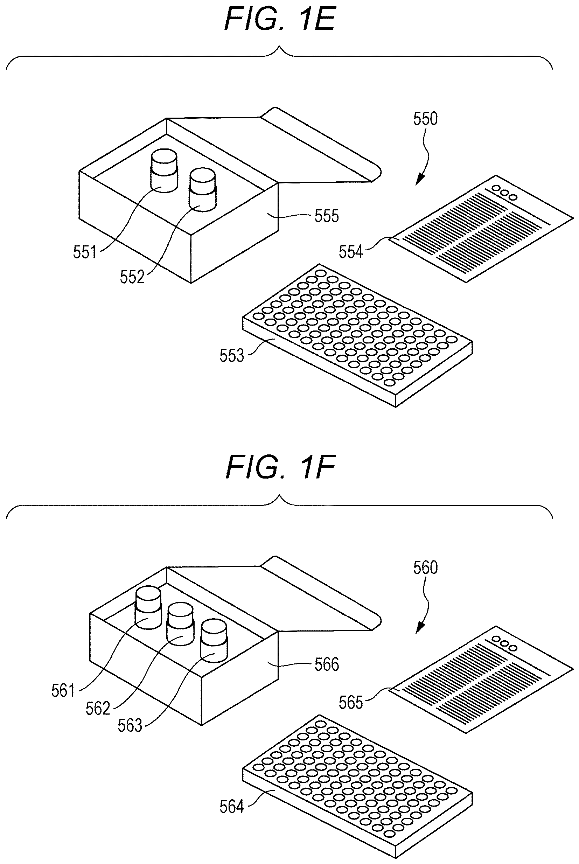

A container storing the above-described various reagents may be packed in a box and provided to the user. This box may contain a package insert of the reagent kit. In this package insert, it is preferable to describe, for example, the constitution of the reagent kit, the protocol for measuring urinary ApoE concentration, and the like. An example of the appearance of the reagent kit as described herein is shown in Fig. 1A. In the figure, 510 denotes a reagent kit, 511 denotes a first container storing a first capture body, 512 denotes a second container storing a second capture body, 513 denotes a third container storing an enzyme as a labeling substance, 514 denotes a fourth container storing a substrate of the enzyme, 515 denotes a package insert, and 516 denotes a packing box.

The reagent kit may further contain a pretreatment liquid for pretreating urine collected from a subject. As the pretreatment liquid, the above-mentioned buffer solution is preferable. The pretreatment liquid may contain a surfactant for solubilizing cells that incorporate ApoE. The details of the buffer and surfactant are the same as those described in the explanation of the diagnosis assisting method above. It is preferable that the first capture body, the second capture body, the labeling substance, the substrate and the pretreatment liquid be stored in separate containers or individually packaged. An example of the appearance of a reagent kit further containing a pretreatment liquid is shown in Fig. 1B. The details of the solid phase are the same as those described in the explanation of the diagnosis assisting method above. In the figure, 520 denotes a reagent kit, 521 denotes a first container storing a first capture body, 522 denotes a second container storing a second capture body, 523 denotes a third container storing an enzyme as a labeling substance, 524 denotes a fourth container storing a substrate of the enzyme, 525 denotes a fifth container storing a pretreatment liquid, 526 denotes a package insert, and 527 denotes a packing box.

The reagent kit may further contain a solid phase for immobilizing the first capture body. In this case, it is preferable that the first capture body, the second capture body, the labeling substance, the substrate and the solid phase be stored in separate containers or individually packaged. An example of the appearance of a reagent kit further containing a solid phase is shown in Fig. 1C. The details of the solid phase are the same as those described in the explanation of the diagnosis assisting method above. In the figure, 530 denotes a reagent kit, 531 denotes a first container storing a first capture body, 532 denotes a second container storing a second capture body, 533 denotes a third container storing an enzyme as a labeling substance, 534 denotes a fourth container storing a substrate of the enzyme, 535 denotes a solid phase (96-well microplate), 536 denotes a package insert, and 537 denotes a packing box. An example of the appearance of a reagent kit further containing a pretreatment liquid is shown in Fig. ID. In the figure, 540 denotes a reagent kit, 541 denotes a first container storing a first capture body, 542 denotes a second container storing a second capture body, 543 denotes a third container storing an enzyme as a labeling substance, 544 denotes a fourth container storing a substrate of the enzyme, 545 denotes a fifth container storing a pretreatment liquid, 546 denotes a solid phase (96-well microplate), 547 denotes a package insert, and 548 denotes a packing box.

The first capture body may be previously immobilized on a solid phase. The labeling substance may be previously bound to the second capture body. In this case, a reagent kit contains a solid phase on which a first capture body is immobilized, a second capture body to which a labeling substance is bound, and a substrate. An example of the appearance of the reagent kit is shown in Fig. IE. In the figure, 550 denotes a reagent kit, 551 denotes a first container storing a second capture body to which an enzyme as a labeling substance is bound, 552 denotes a second container storing a substrate of the enzyme, 553 denotes a solid phase (96-well microplate) on which a first capture body is immobilized, 554 denotes a package insert, and 555 denotes a packing box. An example of the appearance of a reagent kit further containing a pretreatment liquid is shown in Fig. IF. In the figure, 560 denotes a reagent kit, 561 denotes a first container storing a second capture body to which an enzyme as a labeling substance is bound, 562 denotes a second container storing a substrate of the enzyme, 563 denotes a third container storing a pretreatment liquid, 564 denotes a solid phase (96-well microplate) on which a first capture body is immobilized, 565 denotes a package insert, and 566 denotes a packing box.

When the reagent kit is used in the method of assisting diagnosis of the risk of progression to stage 2 or higher diabetic nephropathy, based on the ratios of the value of the urinary ApoE concentration and the values of the urinary concentrations of other nephropathy markers, the kit may further contain a third capture body that specifically binds to other nephropathy markers for capturing the other nephropathy markers and a fourth capture body that specifically binds to other nephropathy markers for detecting the other nephropathy markers.

In addition to the various reagents described above, the reagent kit may appropriately contain a washing liquid of the solid phase, an enzyme reaction terminating agent, a calibrator, and the like.

The present disclosure also includes the use of one or more of the above-described reagents for the manufacturing of a reagent kit for diagnosing the risk of progression to stage 2 or higher diabetic nephropathy. That is, the present disclosure also relates to a use of a first capture body that specifically binds to ApoE for capturing ApoE, a second capture body that specifically binds to ApoE for detecting ApoE, and a labeling substance, for manufacturing a reagent kit for diagnosing the risk of progression to stage 2 or higher diabetic nephropathy.

The present disclosure also includes a marker for assisting diagnosis of risk of progression to stage 2 or higher diabetic nephropathy, which is the ratios of the value of urinary ApoE concentration and the values of the concentrations of other nephropathy markers.

The present disclosure includes an apparatus suitable for acquiring data for assisting diagnosis of the risk of progression to stage 2 or higher diabetic nephropathy. The present disclosure also includes a computer program product for making the computer acquire data for assisting diagnosis of the risk of progression to stage 2 or higher diabetic nephropathy. The medium included in the computer program product may be a computer-readable medium in which a computer program is non-temporarily recorded. More particularly, the disclosure provides a computer readable medium, which is configured, when run on a computer, to carry out the methods of the invention described above. Similarly, the disclosure provides a computer program product adapted for enabling a computer to assist in the diagnosis of the risk of progression to a higher stage diabetic nephropathy, said computer program product comprising a computer readable medium and software instructions, on the computer readable medium for enabling the computer to perform predetermined operations of the method described herein, more particularly for carrying out the determining step based on the values obtained from the measuring step of the methods described herein.

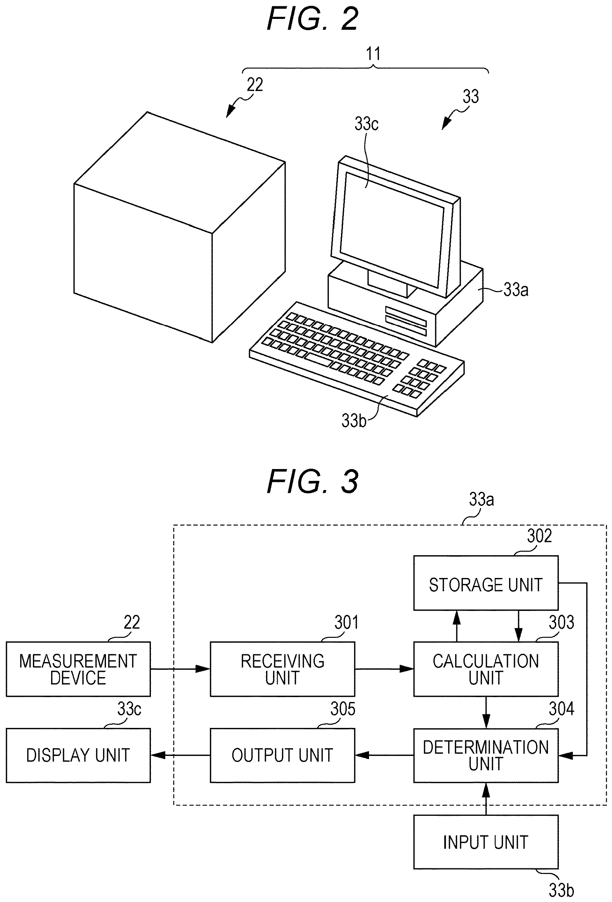

Hereinafter, an example of an apparatus suitable for implementing the above-described method will be described with reference to the drawings. However, the present disclosure is not limited to only the embodiment shown in this example. Fig. 2 is a schematic view of a progression risk diagnosis assisting apparatus for acquiring data for assisting diagnosis of the risk of progression to stage 2 or higher diabetic nephropathy. The progression risk diagnosis assisting apparatus 11 shown in Fig. 2 includes a measurement device 22 and a determination device 33 connected to the measurement device 22.

In the apparatus provided herein, the type of measurement device is not particularly limited, and it can be appropriately selected according to the method of measuring ApoE. In the example shown in Fig. 2, the measurement device 22 is a plate reader capable of detecting a signal generated by an ELISA method using a labeled antibody. The plate reader is not particularly limited as long as it can detect a signal based on the labeling substance used for measuring the marker, and it can be appropriately selected according to the type of the labeling substance. In the example described below, the signal is optical information such as a chemiluminescent signal or a fluorescence signal.

When the plate subjected to an antigen-antibody reaction is set in the measurement device 22, the measurement device 22 acquires optical information based on the labeled antibody specifically bound to ApoE, and the measurement device 22 transmits the obtained optical information to the determination device 33. The optical information acquired by the measurement device 22 also includes information on a specimen whose concentration is known for calculation of the value of the urinary ApoE concentration and the like.

The determination device 33 includes a computer main body 33a, an input unit 33b, and a display unit 33c that displays specimen information, determination results, and the like. The determination device 33 receives the optical information from the measurement device 22. Then, a processor of the determination device 33 executes a program for acquiring data for assisting diagnosis of the risk of progression to stage 2 or higher diabetic nephropathy, based on the optical information.

Fig. 3 is a block diagram showing a software of the computer main body 33a of the progression risk diagnosis assisting apparatus 11 by functional blocks. As shown in Fig. 3, the determination device 33 includes a receiving unit 301, a storage unit 302, a calculation unit 303, a determination unit 304, and an output unit 305. The receiving unit 301 is communicably connected to the measurement device 22 via a network.

The receiving unit 301 receives the information transmitted from the measurement device 22. The storage unit 302 stores an expression for calculating a threshold value required for determination and a density value of each marker, a processing program, and the like. Using the information acquired by the receiving unit 301, the calculation unit 303 calculates the value of urinary ApoE concentration or the ratios of the value of the urinary ApoE concentration and the values of the urinary concentrations of other nephropathy markers, according to the expression stored in the storage unit 302. The determination unit 304 determines whether the value of urinary ApoE concentration or the ratios of the value of the urinary ApoE concentration and the values of the urinary concentrations of other nephropathy markers calculated by the calculation unit 303 is equal to or greater than or less than the threshold value stored in the storage unit 302. The output unit 305 outputs the determination result of the determination unit 304 as data for assisting diagnosis of the risk of progression to stage 2 or higher diabetic nephropathy.

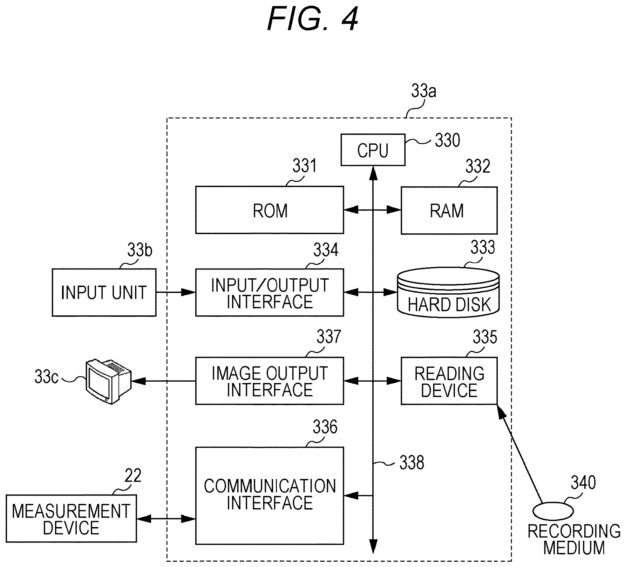

Fig. 4 is a block diagram showing a hardware configuration of the computer main body 33a shown in Fig. 3. As shown in Fig. 4, the computer main body 33a includes a CPU (Central Processing Unit) 330, a ROM (Read Only Memory) 331, a RAM (Random Access Memory) 332, a hard disk 333, an input/output interface 334, a reading device 335, a communication interface 336, and an image output interface 337. The CPU 330, the ROM 331, the RAM 332, the hard disk 333, the input/output interface 334, the reading device 335, the communication interface 336 and the image output interface 337 are data-communicably connected by a bus 338.

The CPU 330 can execute a computer program stored in the ROM 331 and a computer program loaded in the RAM 332. Each function shown in Fig. 3 is executed by the CPU 330 executing an application program. Accordingly, the determination device 33 functions as a device for acquiring data for assisting diagnosis of the risk of progression to stage 2 or higher diabetic nephropathy.

The ROM 331 is constituted of a mask ROM, a PROM, an EPROM, an EEPROM, and the like. In the ROM 331, a computer program executed by the CPU 330 and data used therefor are recorded.

The RAM 332 is constituted of an SRAM, a DRAM, and the like. The RAM 332 is used for reading the computer program recorded in the ROM 331 and the hard disk 333. The RAM 332 is also used as a work area of the CPU 330 when executing these computer programs.

The hard disk 333 has installed therein an operating system for making the CPU 330 execute, a computer program such as an application program (a computer program for acquiring data for assisting diagnosis of the risk of progression to stage 2 or higher diabetic nephropathy) and data used for executing the computer program.

The reading device 335 is constituted of a flexible disk drive, a CD-ROM drive, a DVD-ROM drive, and the like. The reading device 335 can read a computer program or data recorded on a portable recording medium 340.

The input/output interface 334 is constituted of, for example, a serial interface such as USB, IEEE1394 or RS-232C, a parallel interface such as SCSI, IDE or IEEE1284, and an analog interface including a D/A converter, an A/D converter and the like. The input unit 33b such as a keyboard and a mouse is connected to the input/output interface 334. The operator can input various commands and data to the computer main body 33a through the input unit 33b.

The communication interface 336 is, for example, an Ethernet (registered trademark) interface or the like. The computer main body 33a can transmit print data to a printer or the like through the communication interface 336. The measurement device 22 can transmit measurement data (optical information) to the determination device 33 through the communication interface 336.

The image output interface 337 is connected to the display unit 33c constituted of an LCD, a CRT, and the like. Accordingly, the display unit 33c can output the video signal corresponding to the image data given from the CPU 330. The display unit 33c displays an image (screen) according to the inputted video signal.

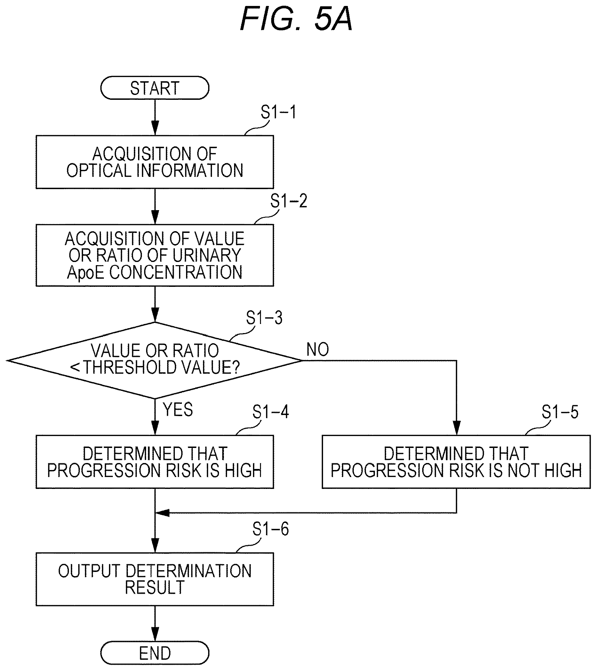

Next, a processing procedure of data acquisition assisting diagnosis of the risk of progression to stage 2 or higher diabetic nephropathy by the progression risk diagnosis assisting apparatus 11 will be described. Fig. 5A is an example of a flowchart for data acquisition assisting diagnosis of the progression risk. A case where the value of urinary ApoE concentration or the ratios of the value of urinary ApoE concentration to the values of the urinary concentrations of other nephropathy markers is acquired from the optical information based on the labeled antibody specifically bound to ApoE and the like, and a determination is made using the obtained value or ratio will be described as an example. However, the present disclosure is not limited to this example.

First, in Step S1-1, the receiving unit 301 of the progression risk diagnosis assisting apparatus 11 receives optical information from the measurement device 22. Next, in Step S1-2, the calculation unit 303 calculates the value of urinary ApoE concentration or the ratios of the value of urinary ApoE concentration to the values of the urinary concentrations of other nephropathy markers, according to the expression for calculating the value of urinary ApoE concentration and the like stored in the storage unit 302, from the optical information received by the receiving unit 301.

Thereafter, in Step S1-3, the determination unit 304 determines whether the value or ratio calculated in Step S1-3 is less than or equal to or greater than the threshold value stored in the storage unit 302. When the calculated value or ratio is less than the threshold value, the routine progresses to Step S1-4, and the determination unit 304 transmits to the output unit 305 a determination result indicating that the risk of progression to stage 2 or higher diabetic nephropathy is high. When the calculated value or ratio is equal to or greater than the threshold value, the routine progresses to Step S1-5, and the determination unit 304 transmits to the output unit 305 a determination result indicating that the risk of progression to stage 2 or higher diabetic nephropathy is not high.

Then, in Step S1-6, the output unit 305 outputs the determination result and displays it on the display unit 33c. Accordingly, the progression risk diagnosis assisting apparatus 11 can provide doctors and the like with data for assisting diagnosis of the risk of progression to stage 2 or higher diabetic nephropathy.

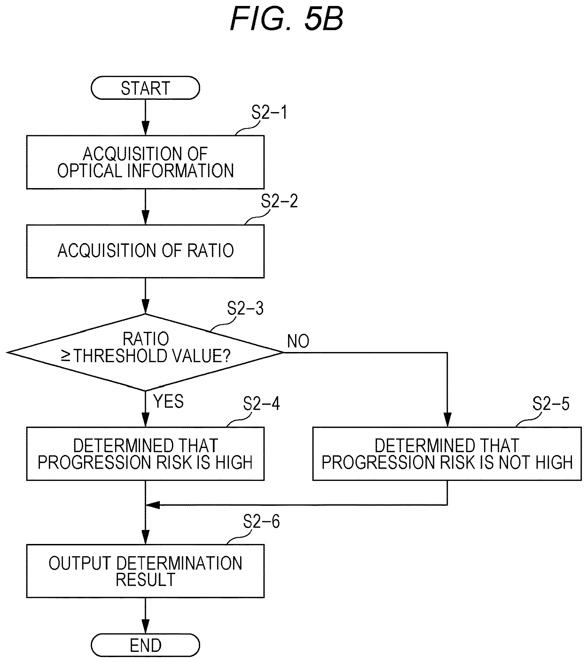

When the ratios of the values of the urinary concentrations of other nephropathy markers to the value of urinary ApoE concentration is acquired and the determination is made using the obtained ratios, it can be made in the same manner as in the description on Steps S 1-1 to S1-6 above, except that the phrase "the value of urinary ApoE concentration or the ratios of the value of urinary ApoE concentration to the values of the urinary concentrations of other nephropathy markers" shall be deemed to be replaced with "the ratios of the values of the urinary concentrations of other nephropathy markers to the value of urinary ApoE concentration", and the phrase "less than" shall be deemed to be replaced with "equal to or greater than" and the phrase "equal to or greater than" shall be deemed to be replaced with "less than". Fig. 5B shows an example of a flowchart for data acquisition assisting diagnosis of the progression risk in this case.

Hereinafter, the present disclosure will be described in detail by examples, but the present disclosure is not limited to these examples.

### EXAMPLES

### EXAMPLES

Blood and urine were collected from subjects who were diabetic patients with stage 1 (the first stage) diabetic nephropathy, and these subjects were divided into a group with progressed diabetic nephropathy and a group without progressed diabetic nephropathy after 6 months. The ApoE concentrations in blood and in urine of subjects in each group were compared.

### (1) Collection of sample from subject

Blood and urine were collected from diabetic patients (n = 17) whose urinary albumin/Cr ratio was lower than 30 mg/gCr and whose GFR classification was 30 mL/min/1.73 m² or more.

### (2) Measurement of biomarker

### (2.1) Measurement of serum creatinine concentration

The serum creatinine (sCR) concentration was measured from blood of each of the subjects, using ELSYSTEM • CRE (Sysmex Corporation) and CRE standard solution (Sysmex Corporation). Specific operations were performed according to the package insert of the reagent.

### (2.2) Measurement of concentrations of ApoE and creatinine in urine

Urine of each subject obtained in the above (1) and Triton-XlOO/PBS attached to a kit described later were mixed in equal amounts and then diluted 25-fold with a specimen diluent. The urinary concentration of ApoE was measured from diluted urine, using Human Apolipoprotein E ELISA Pro Kit (Cell Biolabs, Inc.). Specific operations were performed according to the package insert of the kit, except for using diluted urine instead of serum. The urinary creatinine concentration was measured from urine of each of the subjects, using ELSYSTEM • CRE (Sysmex Corporation) and CRE standard solution (Sysmex Corporation). Specific operations were performed according to the package insert of the reagent. In order to correct errors due to urine volume, the measured urinary ApoE concentration was converted to concentration per gram of creatinine (µg/gCr), using the urinary creatinine concentration.

### (2-3) Other urinary markers

The urinary concentrations of Kidney injury molecule-1 (KIM-1), N-acetylglucosaminidase (NAG), urinary cholesterol (uCHO) and lipocalin-2 (NGAL) were measured as other urinary biomarkers of kidney disease. The KIM-1 concentration was measured using KIM-1 (human) Elisa Kit (Enzo Life Sciences, Inc.) according to the package insert. The NAG concentration was measured using L type NAG (Wako Pure Chemical Industries, Ltd.) and NAG calibrator (Wako Pure Chemical Industries, Ltd.) according to the package insert. The uCHO concentration was measured according to the method described in Japanese Patent No. 4527925. The NGAL concentration was measured using NGAL (human) Elisa Kit (Enzo Life Sciences Inc.) according to the package insert, except that the urine was diluted 50-fold with a specimen diluent attached to the kit. In order to correct errors due to urine volume, the measured concentration of each biomarker was converted to concentration per gram of creatinine (µg/gCr), using the urinary creatinine concentration.

### (3) Classification of subjects

Blood was periodically collected from each of the subjects during 6 months from the sampling in the item (1) above to obtain a plurality of blood specimens (3 to 6 specimens for each subject). The sCR concentration of each blood specimen was measured. Estimated glomerular filtration rate (eGFR) was calculated from the measured value of sCR obtained, according to the following formula. In the formula, the "Age" is the age of the subject.
eGFR (mL/min/1.73 m²) = 194 × sCr^{-1.094} × Age^{-0.287} (when the subject is a female, eGFR = 194 × sCr^{-0.739} × Age^{-0.287})

Taking the eGFR value at the time of sampling as 100%, the eGFR on each blood sampling day during subsequent 6 months was divided. The obtained value was plotted for each elapsed day from the urine collection day. For each plot, the slope of the regression equation passing through the starting point (0th day, 100%) was taken as a progression index. A subject with a progression index obtained of 0 or more was classified into a group in which the eGFR had not decreased (hereinafter, also referred to as "non-progression group" (n = 8)), and a subject with a progression index of less than 0 was classified into a group in which the eGFR had decreased (hereinafter, also referred to as "progression group" (n = 9)).

### (4) Statistical analysis

From the value of urinary biomarker, whether or not the value of the progression index after 6 months (inclination of a change of eGFR value during 6 months) can be predicted was evaluated by single regression analysis.

### (5) Result

The analysis results are shown in Table 1. In the table, the "no ratio" represents the result of a single regression analysis of the single concentration of each biomarker, and the "ApoE ratio" represents the result of a single regression analysis of the ratio of the value of the known nephropathy marker concentration to the value of ApoE concentration. In Table 1, it can be said that there is a significant difference when the risk rate P value is lower than 0.05.

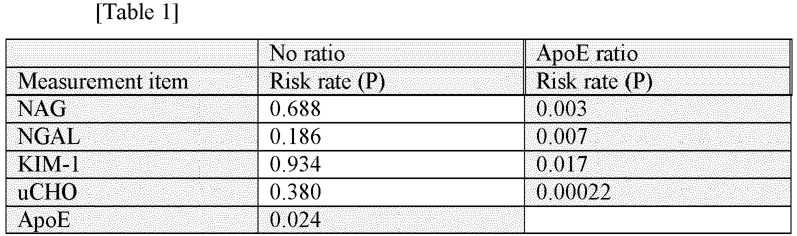

As is apparent from Table 1, it was shown that the change amount of eGFR after 6 months can be predicted from the urinary ApoE concentration, and the progression risk can be accurately predicted. It was shown that the progression risk can be more accurately predicted by using the ratios of the values of the concentrations of other nephropathy markers to the value of urinary ApoE concentration.

## Claims

1. A method for assisting diagnosis of risk of progression to stage 2 or higher diabetic nephropathy, comprising
measuring the urinary apolipoprotein E (ApoE) concentration and optionally the urinary concentration of a nephropathy marker other than the urinary ApoE of a diabetic patient with stage 1 diabetic nephropathy, and
determining whether the patient has a high risk of progressing to stage 2 or higher diabetic nephropathy based on a result of the measurement step,
wherein the patient is determined to have a high risk of progression to stage 2 or higher diabetic nephropathy when:
- the value of urinary ApoE concentration is less than a predetermined threshold value, or
- the ratio of the value of urinary ApoE concentration to the value of the urinary concentration of said other nephropathy marker is less than a predetermined threshold value, or
- the ratio of the value of the urinary concentration of said other nephropathy marker to the value of urinary ApoE concentration is equal to or greater than the predetermined threshold value; and wherein the patient is determined to have a low risk of progression to stage 2 or higher diabetic nephropathy when:
- the value of urinary ApoE concentration is equal to or greater than a predetermined threshold value, or
- the ratio of the value of urinary ApoE concentration to the value of the urinary concentration of said other nephropathy marker is equal to or greater than a predetermined threshold value, or
- the ratio of the value of the urinary concentration of said other nephropathy marker to the value of urinary ApoE concentration is less than the predetermined threshold value.

2. The method according to claim 1, wherein said other nephropathy marker is selected from the group consisting of uTP, uALB, α1-M, NAG, L-FABP, NGAL, KIM- 1, uCHO, type IV collagen, ApoA1 and ApoB.

3. The method according to any one of claims 1 or 2, wherein said other nephropathy marker is selected from the group consisting of uALB, NAG, NGAL, KIM-1, uCHO and ApoA1.

4. An apparatus for acquiring data for assisting diagnosis of the risk of progression to stage 2 or higher diabetic nephropathy, comprising
a computer containing a processor and a memory under control of the processor,
wherein the memory stores a computer program for making the computer execute the following steps:
measuring urinary apolipoprotein E (ApoE) concentration of a diabetic patient with stage 1 diabetic nephropathy and optionally the urinary concentration of a nephropathy marker other than the urinary ApoE, and
determining whether the patient has a high risk of progressing to stage 2 or higher diabetic nephropathy based on a result of the measurement step,
wherein the patient is determined to have a high risk of progression to stage 2 or higher diabetic nephropathy when:
- the value of urinary ApoE concentration is less than a predetermined threshold value, or
- the ratio of the value of urinary ApoE concentration to the value of the urinary concentration of said other nephropathy marker is less than a predetermined threshold value, or
- the ratio of the value of the urinary concentration of said other nephropathy marker to the value of urinary ApoE concentration is equal to or greater than the predetermined threshold value, and wherein the patient is determined to have a low risk of progression to stage 2 or higher diabetic nephropathy when:
- the value of urinary ApoE concentration is equal to or greater than a predetermined threshold value, or
- the ratio of the value of urinary ApoE concentration to the value of the urinary concentration of said other nephropathy marker is equal to or greater than a predetermined threshold value, or
- the ratio of the value of the urinary concentration of said other nephropathy marker to the value of urinary ApoE concentration is less than the predetermined threshold value.

## Patentansprüche

1. Verfahren zur Unterstützung der Diagnose des Risikos des Fortschreitens zu diabetischer Nephropathie der Stufe 2 oder höher, umfassend:
Messen der Harnkonzentration von Apolipoprotein E (ApoE) und gegebenenfalls der Harnkonzentration eines von dem Harn-ApoE verschiedenen Nephropathiemarkers eines diabetischen Patienten mit diabetischer Nephropahthie der Stufe 1, und
Bestimmen, ob der Patient ein hohes Risiko des Fortschreitens zu diabetischer Nephropathie der Stufe 2 oder höher aufweist, auf Grundlage eines Ergebnisses des Messschritts,
wobei bestimmt wird, dass der Patient ein hohes Risiko des Fortschreitens zu diabetischer Nephropathie der Stufe 2 oder höher aufweist, wenn:
- der Wert der Harn-ApoE-Konzentration kleiner als ein vorbestimmter Schwellenwert ist, oder
- das Verhältnis des Werts der Harn-ApoE-Konzentration zu dem Wert der Harnkonzentration des anderen Nephropathiemarkers kleiner als ein vorbestimmter Schwellenwert ist, oder
- das Verhältnis des Werts der Harnkonzentration des anderen Nephropathiemarkers zu dem Wert der Harn-ApoE-Konzentration gleich dem oder größer als der vorbestimmte Schwellenwert ist; und
wobei bestimmt wird, dass der Patient ein niedriges Risiko des Fortschreitens zu diabetischer Nephropathie der Stufe 2 oder höher aufweist, wenn:
- der Wert der Harn-ApoE-Konzentration gleich einem oder größer als ein vorbestimmter Schwellenwert ist, oder
- das Verhältnis des Werts der Harn-ApoE-Konzentration zu dem Wert der Harnkonzentration des anderen Nephropathiemarkers gleich einem oder größer als ein vorbestimmter Schwellenwert ist, oder
- das Verhältnis des Werts der Harnkonzentration des anderen Nephropathiemarkers zu dem Wert der Harn-ApoE-Konzentration kleiner als der vorbestimmte Schwellenwert ist.

2. Verfahren gemäß Anspruch 1, wobei der andere Nephropathiemarker ausgewählt ist aus der Gruppe bestehend aus uTP, uALB, α1-M, NAG, L-FABP, NGAL, KIM-1, uCHO, Typ-IV-Collagen, ApoA1 und ApoB.

3. Verfahren gemäß einem der Ansprüche 1 oder 2, wobei der andere Nephropathiemarker ausgewählt ist aus der Gruppe bestehend aus uALB, NAG, NGAL, KIM-1, uCHO und ApoA1.

4. Vorrichtung zur Erfassung von Daten zur Unterstützung der Diagnose des Risikos des Fortschreitens zu diabetischer Nephropathie der Stufe 2 oder höher, umfassend:
einen Computer, der einen Prozessor und einen Speicher unter der Steuerung des Prozessors enthält,
wobei der Speicher ein Computerprogramm zum Anweisen des Computers, die folgenden Schritte auszuführen, speichert:
Messen der Harnkonzentration von Apolipoprotein E (ApoE) eines diabetischen Patienten mit diabetischer Nephropahthie der Stufe 1 und gegebenenfalls der Harnkonzentration eines von dem Harn-ApoE verschiedenen Nephropathiemarkers, und
Bestimmen, ob der Patient ein hohes Risiko des Fortschreitens zu diabetischer Nephropathie der Stufe 2 oder höher aufweist, auf Grundlage eines Ergebnisses des Messschritts,
wobei bestimmt wird, dass der Patient ein hohes Risiko des Fortschreitens zu diabetischer Nephropathie der Stufe 2 oder höher aufweist, wenn:
- der Wert der Harn-ApoE-Konzentration kleiner als ein vorbestimmter Schwellenwert ist, oder
- das Verhältnis des Werts der Harn-ApoE-Konzentration zu dem Wert der Harnkonzentration des anderen Nephropathiemarkers kleiner als ein vorbestimmter Schwellenwert ist, oder
- das Verhältnis des Werts der Harnkonzentration des anderen Nephropathiemarkers zu dem Wert der Harn-ApoE-Konzentration gleich dem oder größer als der vorbestimmte Schwellenwert ist; und
wobei bestimmt wird, dass der Patient ein niedriges Risiko des Fortschreitens zu diabetischer Nephropathie der Stufe 2 oder höher aufweist, wenn:
- der Wert der Harn-ApoE-Konzentration gleich einem oder größer als ein vorbestimmter Schwellenwert ist, oder
- das Verhältnis des Werts der Harn-ApoE-Konzentration zu dem Wert der Harnkonzentration des anderen Nephropathiemarkers gleich einem oder größer als ein vorbestimmter Schwellenwert ist, oder
- das Verhältnis des Werts der Harnkonzentration des anderen Nephropathiemarkers zu dem Wert der Harn-ApoE-Konzentration kleiner als der vorbestimmte Schwellenwert ist.

## Revendications

1. Procédé pour faciliter le diagnostic de risque de progression vers une néphropathie diabétique de stade 2 ou supérieur, comprenant
la mesure de la concentration d'apolipoprotéine E (ApoE) urinaire et, facultativement, de la concentration urinaire d'un marqueur de néphropathie autre que l'ApoE urinaire d'un patient diabétique atteint de néphropathie diabétique de stade 1, et
la détermination du fait que le patient présente un risque élevé de progression vers une néphropathie diabétique de stade 2 ou supérieur sur la base d'un résultat de l'étape de mesure,
dans lequel il est déterminé que le patient présente un risque élevé de progression vers une néphropathie diabétique de stade 2 ou supérieur lorsque :
- la valeur de concentration urinaire d'ApoE est inférieure à une valeur de seuil prédéterminée, ou
- le rapport de la valeur de concentration urinaire d'ApoE à la valeur de la concentration urinaire dudit autre marqueur de néphropathie est inférieur à une valeur de seuil prédéterminée, ou
- le rapport de la valeur de la concentration urinaire dudit autre marqueur de néphropathie à la valeur de concentration urinaire d'ApoE est supérieur ou égal à la valeur de seuil prédéterminée ; et
dans lequel il est déterminé que le patient présente un faible risque de progression vers une néphropathie diabétique de stade 2 ou supérieur lorsque :
- la valeur de concentration urinaire d'ApoE est supérieure ou égale à une valeur de seuil prédéterminée, ou
- le rapport de la valeur de concentration urinaire d'ApoE à la valeur de la concentration urinaire dudit autre marqueur de néphropathie est supérieur ou égal à une valeur de seuil prédéterminée, ou
- le rapport de la valeur de la concentration urinaire dudit autre marqueur de néphropathie à la valeur de concentration urinaire d'ApoE est inférieur à la valeur de seuil prédéterminée.

2. Procédé selon la revendication 1, dans lequel ledit autre marqueur de néphropathie est choisi dans le groupe constitué de uTP, uALB, α1-M, NAG, L-FABP, NGAL, KIM-1, uCHO, collagène de type IV, ApoA1 et ApoB.

3. Procédé selon l'une quelconque des revendications 1 ou 2, dans lequel ledit autre marqueur de néphropathie est choisi dans le groupe constitué de uALB, NAG, NGAL, KIM-1, uCHO et ApoA1.

4. Appareil d'acquisition de données pour faciliter le diagnostic du risque de progression vers une néphropathie diabétique de stade 2 ou supérieur, comprenant un ordinateur contenant un processeur et une mémoire sous le contrôle du processeur,
dans lequel la mémoire stocke un programme informatique pour amener l'ordinateur à exécuter les étapes suivantes :
mesure de la concentration urinaire d'apolipoprotéine E (ApoE) d'un patient diabétique atteint de néphropathie diabétique de stade 1 et, facultativement, de la concentration urinaire d'un marqueur de néphropathie autre que l'ApoE urinaire, et
détermination du fait que le patient présente un risque élevé de progression vers une néphropathie diabétique de stade 2 ou supérieur sur la base d'un résultat de l'étape de mesure,
dans lequel il est déterminé que le patient présente un risque élevé de progression vers une néphropathie diabétique de stade 2 ou supérieur lorsque :
- la valeur de concentration urinaire d'ApoE est inférieure à une valeur de seuil prédéterminée, ou
- le rapport de la valeur de concentration urinaire d'ApoE à la valeur de la concentration urinaire dudit autre marqueur de néphropathie est inférieur à une valeur de seuil prédéterminée, ou
- le rapport de la valeur de la concentration urinaire dudit autre marqueur de néphropathie à la valeur de concentration urinaire d'ApoE est supérieur ou égal à la valeur de seuil prédéterminée ; et
dans lequel il est déterminé que le patient présente un faible risque de progression vers une néphropathie diabétique de stade 2 ou supérieur lorsque :
- la valeur de concentration urinaire d'ApoE est supérieure ou égale à une valeur de seuil prédéterminée, ou
- le rapport de la valeur de concentration urinaire d'ApoE à la valeur de la concentration urinaire dudit autre marqueur de néphropathie est supérieur ou égal à une valeur de seuil prédéterminée, ou
- le rapport de la valeur de la concentration urinaire dudit autre marqueur de néphropathie à la valeur de concentration urinaire d'ApoE est inférieur à la valeur de seuil prédéterminée.
